# EUROPEAN PATENT SPECIFICATION

(11) **EP 0 347 433 B1**
(45) Date of publication and mention of the grant of the patent: **02.03.1994**
(21) Application number: 88907749.1
(22) Date of filing: 05.09.1988
(51) Int. Cl.: C12P 21/08, C12N 15/13

(54) **RECOMBINANT ANTIBODY**
REKOMBINANT-ANTIKÖRPER
ANTICORPS RECOMBINANT

(30) Priority: 04.09.1987 GB 8720833
(43) Date of publication of application: 27.12.1989
(73) Proprietor: CELLTECH LIMITED, Slough Berkshire SL1 4EN (GB)
(72) Inventor: BODMER, Mark, William, Henley-on-Thames Oxfordshire R619 1DJ (GB); ADAIR, John, Robert, Stokenchurch High Wycombe HP14 3RN (GB); WHITTLE, Nigel, Richard, Cobham Surrey KT11 2LF (GB)
(74) Representative: Mercer, Christopher Paul
(86) International application number: PCT/GB88/00730
(87) International publication number: WO 89/01974

(56) References cited:
- EP-A- 0 146 413
- WO-A-86/01533

## Description

The present invention relates to an altered antibody molecule (AAM) having an altered number of cysteine residues in its hinge region and to a process for its production using recombinant DNA technology.

In the present application:
the term "MAb" is used to denote a monoclonal antibody;
the term "recombinant antibody molecule" (RAM) is used to describe an antibody produced by any process involving the use of recombinant DNA technology, including any analogues of natural immunoglobulins or their fragments; and
the term "humanised antibody molecule" (HAM) is used to describe a molecule having an antigen binding site derived from an immunoglobulin from a non-human species, the remaining immunoglobulin-derived parts of the molecule being derived from a human immunoglobulin. The antigen binding site may comprise either complete variable domains fused onto constant domains or only the complementarity determining regions (CDRs) grafted onto appropriate framework regions in the variable domains.

In the description, reference is made to a number of publications by number. The publications are listed in numerical order at the end of the description.

Natural immunoglobulins have been known for many years, as have the various fragments thereof, such as the Fab, (Fab′)₂ and Fc fragments, which can be derived by enzymatic cleavage. Natural immunoglobulins comprise a generally Y-shaped molecule having an antigen-binding site at the end of each arm. The remainder of the structure, and particularly the stem of the Y, mediates the effector functions associated with immunoglobulins.

At the junction of the arms of the Y-shaped molecule, there is an area known as the hinge region. In this region there are, depending on the class of the antibody, between 2 and 11 inter-heavy chain disulphide bonds. These disulphide bonds are responsible for holding together the two parts of the complete antibody molecule. In a Fab fragment, the hinge region has been enzymatically separated from the antigen binding region. Thus, the Fab fragment comprises a light chain/truncated heavy chain dimer. However, in the (Fab′)₂ fragment, the Fc portion is cleaved off the antigen binding regions on the C-terminal side of the hinge region. Thus, the (Fab′)₂ fragment comprises two light chain/truncated heavy chain dimers held together in a tetrameric structure by the hinge region.

The hinge region allows the arms of the Y-shaped antibody molecule to move relative to one another. It is conjectured that the degree of movement is determined to a large extent by the number of disulphide bonds in the hinge region. It is also believed that the hinge region plays a key role in the transmission of conformational changes from the antigen binding regions to the Fc portion of the molecule. Such conformational changes may be necessary in order to activate the effector functions of the immunoglobulin molecule.

In natural antibodies, in particular the IgGs, the hinge represents a distinct region of the antibody, at both the protein and gene level. High resolution data on the in vivo conformation of the hinge region are not available from the structural studies performed to date. However, model building suggests that the hinge regions may form a relatively simple extended or helix-like structure linked by disulphide bridges.

Natural immunoglobulins and their fragments have been used in diagnosis and, to a more limited extent, in therapy. However, such uses, especially in therapy, have been hindered by the polyclonal nature of natural immunoglobulins. A significant step towards the realisation of the potential of immunoglobulins as therapeutic agents was the discovery of monoclonal antibodies (1) of defined antigen specificity. Most MAbs are produced by fusions of rodent spleen cells with rodent myeloma cells. They are therefore essentially rodent MAbs. There are very few reports of the production of human MAbs.

There have been made proposals for "humanizing" rodent MAbs. These techniques generally involve the use of recombinant DNA technology to manipulate DNA sequences encoding the polypeptide chains of the antibody molecule.
Some early methods for carrying out such a procedure are described in EP-A-0 171 496 (Res. Dev. Corp. Japan), EP-A-0 173 494 (Stanford University), EP-A-0 194 276 (Celltech Limited) and WO-A-8 702 671 (Int. Gen. Eng. Inc.).

In an alternative approach, described in EP-A-87302620.7 (EP-A-239400) (Winter), the complementarity determining regions (CDRs) of a mouse MAb have been grafted onto the framework regions of the variable domains of a human immunoglobulin by site directed mutagenesis using long oligonucleotides.

It has been widely suggested that immunoglobulins, and in particular MAbs, could potentially be very useful in the diagnosis and treatment of cancer (2,3). There has therefore been much activity in trying to produce immunoglobulins or MAbs directed against tumour-specific antigens. So far, over one hundred MAbs directed against a variety of human carcinomas have been used in various aspects of tumour diagnosis or treatment (4).

In our copending, concurrently filed application No. WO89/01783 (PA 149) there is described a humanised antibody molecule (HAM) having an antigen binding site wherein at least the complementarity determining regions (CDRs) of the variable domain are derived from the mouse monoclonal antibody B72.3 (B72.3 MAb) and the remaining immunoglobulin-derived parts of the HAM are derived from a human immunoglobulin, and a process for its production.

The B72.3 MAb is a mouse MAb of the type IgG1-Kappa raised against a membrane-enriched extract of a human liver metastatis of a breast carcinoma (5). The B72.3 MAb has been extensively studied in a number of laboratories. It has been shown to recognise a tumour-associated glycoprotein TAG-72, a mucin-like molecule with a molecular weight of approximately 10⁶ (6). Immunohistochemical studies have demonstrated that the B72.3 MAb recognises approximately 90% of colorectal carcinomas, 85% of breast carcinomas and 95% of ovarian carcinomas. However, it shows no significant cross-reactivity with a wide spectrum of normal human tissues (7 to 10).

It has been suggested, for instance in EP-A-0 194 276, that effector or reporter molecules may be attached to an antibody or a fragment thereof in order to increase its effectiveness as a therapeutic or diagnostic agent. The attachment may be by means of a covalent bridging structure. Alternatively, where the effector or reporter molecule is a protein, it may be coexpressed as the C-terminal part of a fusion protein, the N-terminal part of which comprises at least the variable domain of one of the chains of the antibody molecule or fragment.

In all the work carried out so far, the hinge region, if present, in the antibody molecule or fragment has been that normally associated with the CH1 domain of the antibody molecule. There has been no suggestion that the hinge region should in any way be altered except in so far as it may be necessary to alter its C- or N-terminal sequence to facilitate manipulations by recombinant DNA technology.

According to a first aspect of the present invention, there is provided an altered antibody molecule (AAM) having a hinge region which has a different number of cysteine residues from that found in the hinge region normally associated with the CH1 domain of the antibody molecule, wherein at least one cysteine residue is capable of forming a heavy chain-heavy chain disulphide bond.

The AAM of the present invention may comprise: a complete antibody molecule, having full length heavy and light chains; an (Fab′)₂ fragment; or any other fragment including a hinge region. The antigen binding portions of the AAM may, if desired, have different specificities, the antibody in this case being bispecific.

The AAM of the present invention may have attached to it an effector or reporter molecule. For instance, the AAM may have a macrocycle, for chelating a heavy metal atom, or a toxin, such as ricin, attached to it by a covalent bridging structure. Alternatively, the procedures of recombinant DNA technology may be used to produce a AAM in which the Fc fragment or CH₃ domain of a complete antibody molecule has been replaced by an enzyme or toxin molecule.

Preferably, the AAM of the present invention is also a HAM, for instance of the type described in the above-referenced copending application. The variable domains of the HAM may comprise either the entire variable domains of a rodent MAb or may comprise the framework regions of a human variable domain having grafted thereon the CDRs of the rodent MAb. The remainder of the HAM may be derived from any suitable human immunoglobulin. However, it need not comprise only protein sequences from the human immunoglobulin. For instance, a gene may be constructed in which a DNA sequence encoding part of a human immunoglobulin chain is fused to a DNA sequence encoding the amino acid sequence of a polypeptide effector or reporter molecule.

The altered hinge region of the AAM of the present invention may comprise a complete hinge region derived from an antibody of different class or subclass from that of the CH1 domain. Thus, for instance, a CH1 domain of class γ 1 may be attached to a hinge region of class γ 4. Alternatively, the new hinge region may comprise part of a natural hinge or a repeating unit in which each unit in the repeat is derived from a natural hinge region. In a further alternative, the natural hinge region may be altered by converting one or more cysteine residues into neutral residues, such as alanine, or by converting suitably placed residues into cysteine residues. It can thus be seen that the number of cysteine residues in the hinge region may be increased or decreased.

In one preferred aspect of the invention, the number of cysteine residues in the hinge region is reduced to one. This will have the advantage that it will facilitate assembly of the antibody molecules, particularly bispecific antibody molecules and antibody molecules wherein the Fc portion has been replaced by an effector or reporter molecule, since it will only be necessary to form a single disulphide bond. This will provide a specific target for attaching the hinge region either to another hinge region or to an effector or reporter molecule, either directly or indirectly, by chemical means.

In an alternative preferred aspect, the number of cysteine residues in the hinge is increased. The advantage of this is that it will facilitate the use of the cysteine thiol groups for attaching effector or reporter molecules to the AAM. For instance, ⁹⁹^{m} technecium, a radiolabel, may be attached to hinge cysteines either directly or by use of a macrocycle ligand. Increasing the number of cysteines can also be used to stabilize the interactions between adjacent hinges.

It will be appreciated by the skilled person that the present invention enables him to make an informed trade-off between improved specificity of attachment and assembly on the one hand and improved stability and decreased specificity on the other hand.

Preferably, the AAM of the present invention is produced by recombinant DNA technology. Therefore, according to a second aspect of the present invention, there is provided a process for producing an AAM according to the first aspect of the invention, which process comprises:
(a) producing in an expression vector a transcription unit which includes a DNA sequence encoding an antibody heavy chain having a hinge region which has a different number of cysteine residues from that found in the hinge region normally associated with the CH1 domain of the antibody molecule.

The transcription unit may be produced by splicing a DNA sequence encoding the CH1 region from an antibody of one class to a DNA sequence encoding the hinge region from an antibody of a different class.

Alternatively, the transcription unit may be produced by cloning the CH1 domain and hinge region from an antibody of one class and altering the number of cysteine residue encoding DNA triplets by site directed mutagenesis. Preferably, where the number of cysteine residues is to be decreased, the cysteine-encoding sequences are mutated to alanine-encoding sequences. Where the number of cysteine-encoding sequences is to be increased, any suitably positioned non-cysteine residue may be altered.

Preferably, the process of the second aspect of the invention includes the steps of:
(b) transfecting a cell line with the vector; and
(c) culturing the transfected cell line to produce the AAM.

In the process of the second aspect of the present invention, as the vector encodes only the heavy chain antibody polypeptide it will be necessary to arrange for the cell line to produce a complementary light chain. In order to achieve this, one of three alternative strategies may be employed.

In the first alternative, the cell line may be transfected with a second vector, the second vector encoding a complementary light chain-derived polypeptide. Preferably, the vectors are identical except in so far as the coding sequences and selectable markers are concerned so as to ensure as far as possible that each polypeptide chain is equally expressed.

In the second alternative, the vector may include sequences coding for both light chain- and heavy chain-derived polypeptides.

In the third alternative, it is possible to produce the AAM by using a host cell which naturally secretes a complementary light chain.

The present invention also includes cloning and expression vectors and transfected cell lines used in the process of the invention, therapeutic and diagnostic compositions containing the AAM of the invention and uses of such compositions in therapy and diagnosis.

The general methods by which the vectors may be constructed, transfection methods and culture methods are well known per se and form no part of the invention. Such methods are shown, for instance, in references 11 and 12.

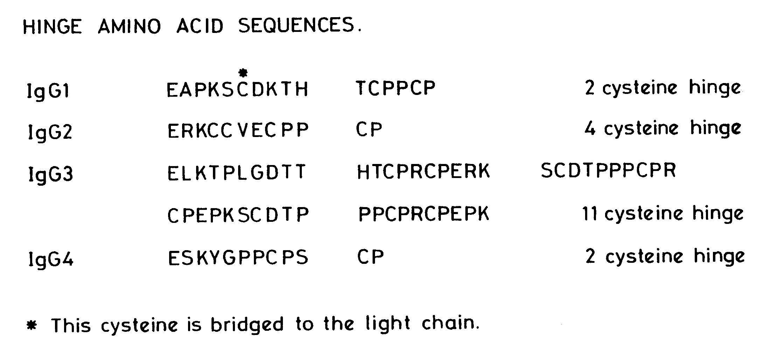

The present invention is now described, by way of example only, with reference to the accompanying drawing which shows the natural amino acid sequences of the four human IgG hinge regions.

In our above-referenced copending, concurrently filed application No. WO89/01783 (PA 149) we describe in detail the production of humanized antibody molecules based on the B72.3 MAb and in particular, the construction of whole antibody molecules and F(ab′) fragments is shown. The application describes the construction of various expression vectors and their use in producing humanised B72.3 antibodies (B72.3 HAMs). This work forms the basis for the work now described.

Natural human IgGs can have one of four possible hinge regions. The amino acid sequences of these hinge regions are shown in the accompanying drawing. From the known amino acid sequence it will readily be possible for the skilled person to design or mutate oligonucleotides to encode these sequences, or variants thereof with cysteine additions or deletions.

### EXAMPLE 1

In Example 3 of the PA 149 application, there is described an (Fab′)₂ molecule having the B72.3 variable domains and a human IgG4 CH1 domain and hinge region. In order to encourage selective reduction at the hinge of this B72.3 HAM and to reduce the complexity of subsequent chemical additions at the hinge, the number of cysteines in the B72.3 HAM hinge region (which is normally two) was reduced to one by replacing the second hinge cysteine residue with an alanine residue.
A part of the amino acid sequence of the hinge region and its corresponding DNA encoding sequence is shown below.

CCC CCA TGC CCA TCA TGC CCA
Pro Pro Cys Pro Ser Cys Pro

### Modified B72.3 Chimaeric IgG4(Fab′)₂

### Single Cysteine IgG4 Hinge

### Construction and Assembly of Modified F(ab′) Gene

To construct a modified hinge onto the end of the CH1 domain in pJA108 (described in our copending application PA149), four oligonucleotides were made which together are able to code for the last five amino acids of the CH1 domain, the hinge sequence, two inframe stop codons and an EcoRI site.

The oligonucleotides required to form the new Cys-Ala modified hinge sequence have the following sequences: 
1
5'TCGACAAGAGACTTGAGTCCAAATATGGG
3'GTTCTCTCAACTCAGGTTTATACCCGGGGG
2

3
5'CCCCCGTGCCCATCAGCCCCATGATG
3'CACGGGTAGTCGGGGTACTACTTAA
4
Oligonucleotides 1 and 3 were used to form the sense strand and oligonucleotides 2 and 4 were used to form the anti-sense strand.

The modified CH1/hinge region junction encoded by the modified gene has the amino acid sequence: 
Lys Val Asp Lys Arg Val Glu Ser Lys Tyr Gly Pro Pro
Cys Pro Ser Ala Pro Stop Stop
The CH1/hinge region junction is between the Val and Glu residues.

The oligonucleotides were assembled and cloned into M13mp11 between the SalI and EcoRI sites in the polylinker, sequenced, reisolated and ligated to the gene containing the EcoRI - SalI 700bp fragment from pJA108 to construct the modified (hinge cys to ala) chimaeric B72.3 F(ab′) heavy chain gene.

### Assembly of Gene in Expression Vector

The modified chimaeric B72.3 F(ab′) heavy chain gene fragment, assembled as described above, was subsequently cloned into the EcoRI vector fragment of pJA96 to give pJA115.

### Test of Genes in COS Cells

The genes were tested in COS cells as described in the PA149 application. On non-reducing SDS-PAGE the material appeared to be produced as F(ab′) material only. Reducing SDS-PAGE the material appeared to be produced as F(ab′) material only. Reducing SDS-PAGE showed the presence of light chain and truncated heavy chain equivalent to that expected from the modified F(ab′) gene.

### Development of Stable Cell Lines in CHO Cells

The expression plasmid pJA115, comprising the hinge modified B72.3 chimaeric F(ab′) heavy chain gene fragment expressed from the HCMV promoter, was introduced by electroporation into the CHO cell line cL18 described in the PA149 application. The procedure was similar to that described for introduction of the full length chimaeric heavy chain except that the SalI digestion was omitted and the DNA was introduced as closed circular DNA. Cell lines resistant to mycophenolic acid and expressing functional modified F(ab′) antibody was identified by screening culture supernatants in an antigen binding ELISA format assay as described earlier. Cell lines expressing between 0.1-10 µg/ml modified F(ab′) were isolated. One cell line, 18ΔF9, was used for further studies.

CHO cell lines expressing the modified F(ab′) were also isolated using gene amplification. A plasmid vector comprising the chimaeric light chain and modified F(ab′) heavy chain gene fragment, each expressed separately from the HCMV promoter, and the glutamine synthetase (GS) minigene described in our International patent application No. PCT/GB88/00602 (EP-A-323097), namely pEE6.cH.cL.GS, was introduced into CHO-K1 cells by the calcium phosphate precipitation procedure.

Transfectants were selected in GMEM medium as described in our International patent application No. PCT/GB88/00602 (EP-A-323097) comprising 20 µM methionine sulphoximine (MSX). Cell lines expressing functional modified F(ab′) antibody were identified by screening culture supernatants in an antigen binding ELISA format assay as described in our copending application. Cell lines expressing between 0.05-1µg/ml modified F(ab′) were obtained and subjected to screening for gene amplification by plating out in MSX concentrations ranging from 30-5000µM. Amplified cell lines growing in medium containing MSX at 200µM were found to express modified F(ab′) to a level of 10-20µg/ml. One amplified cell line, 31.2 was used for further studies.

### Purification of modified chimaeric F(ab′)₂ Antibody

Hinge modified chimaeric F(ab′) was purified from CHO cell supernatants using standard procedures. CHO cell culture supernatant containing modified chimaeric F(ab′) was adjusted to 60% ammonium sulphate, the precipitate was resuspended in suitable buffer and passed over a DEAE-Sepharose® column. Fractions containing F(ab′) material were pooled and, after dialysis, passed over S-Sepharose®. Fractions containing F(ab′) material were pooled and then dialysed extensively into PBS and concentrated by ultrafiltration. By this process, the yield of F(ab′)₂ is increased to approximately 40% of the finally purified material.

Hinge modified F(ab′) was purified from CHO cell supernatant using immunopurification. An immunopurification reagent was prepared by linking NH3/41, an antibody with specificity for human Kappa chain sequence, to cyanogen bromide activated Sepharose® by standard methodology. This material was packed into a column and equilibrated with PBS. CHO cell culture supernatant containing modified chimaeric F(ab′) was applied to the column and the column was washed with PBS. Elution of modified chimaeric F(ab′) was then achieved using 4.5M guanidine hydrochloride. Fractions containing modified chimaeric F(ab′) were then dialyzed extensively into PBS and concentrated by ultrafiltration. In repeat purifications, approximately 10% of the material can be found as F(ab′ )₂ which forms without further treatment.

### Crosslinking of Modified Chimaeric F(ab′)₂ Antibody

F(ab′)₂ material from mouse B72.3, prepared by enzymatic digestion of whole antibody, and modified chimaeric F(ab′) material prepared as described in the preceeding paragraph were chemically crosslinked by linking half-cysteine residues via thioether bonds with the use of the homo-bifunctional cross-linking reagent 1,6 bismaleimidohexane (Sigma). B72.3 F(ab′)₂ or modified chimaeric F(ab′) at 1-5 mg/ml in 0.15M phosphate buffer pH8.0, containing 2mM EDTA, was reduced by the addition of 2-mercaptoethylamine to a final concentration of 5mM and incubated at 37°c for 30 minutes. After reduction, samples were desalted on a Sephadex® G25 column equilibrated with 0.1M citric acid/0.2M Na₂PO₄ pH6.0/2mM EDTA. The crosslinker was dissolved in dimethylformamide in a final concentration of 72mM and added to the freshly reduced F(ab′)SH at a level of 1.9mM (approx. 22 fold excess over titratable thiols) and incubated for 90 minutes with constant mixing. N-ethyl maleimide was added to a final concentration of 9mM and incubated further for 30 minutes before desalting into 0.1M citric acid/0.2M Na₂PO₄ pH6.0/2mM EDTA. The maleimidated F(ab′), (Fab′)(MAL), was immediately added to a fresh batch of F(ab′) SH at a molar ratio of 1.1:1.0 and incubated at room temperature with constant mixing for 20 hours.

The composition of the crosslinking reaction was determined by HPLC gel filtration after overnight incubation. 10µl of the reaction mixture was added to 10µl of 200mM 2-mercaptoethylamine and incubated for 15 minutes. A further addition of 20µl of 800mM iodoacetamide was made and incubated for 15 minutes. The reduced and alkylated sample was then analysed by HPLC GF 250 and the percentage composition of the chromatogram was determined by integration. Material eluting in the position of F(ab′)₂ was assumed to be chemically crosslinked F(ab′)₂. The elution times closely matched the expected F(ab′)₂ retention times of various standards. The percentage crosslinking was higher for the chimaeric F(ab′) material (one cysteine in the hinge) than for the mouse F(ab′) material (three cysteines in the hinge) and contained less aggregated material. Reduction in the complexity of the hinge in this way may therefore lead to more efficient linking and superior product.

### EXAMPLE 2

### Chimaeric B72.3 IgG4 F(ab′)₂ (IgG1 Hinge)

### Construction of F(ab′) Heavy Chain Gene

To construct a hinge modified gene, the chimaeric F(ab) region containing the B72.3VH/IgG4 gene was isolated as a 0.7 kbp fragment from JA 108 (described in our copending patent application PA 149) by treating the DNA with SalI, removing the 5′ phosphate from the SalI site with calf intestinal phosphatase (CIP), and recutting the DNA with EcoRI.

The IgG1 hinge was assembled by preparing appropriate oligonucleotides to encode the IgG1 hinge. 500 pm of top and bottom strand oligonucleotide were kinase labelled and annealed by heating to 70°C and cooling to room temperature in the kinase buffer. The hinge fragments were ligated to the 0.7 kbp fragment from JA108 prepared as above, and the CIP′ed 5′ ends were kinased.

### Assembly of Gene in Expression Vector

The chimaeric B72.3 F(ab′) heavy chain gene fragment, assembled as described above, was subsequently cloned into the EcoRI/CIP treated vector of pJA96 to give TR001. Expression of TR001 in suitable cells with an expression vector capable of producing a useful light chain, for example chimaeric or humanised B72.3, will produce material which will assemble to give F(ab′) and which will on suitable post translational modification in vivo or in vitro give F(ab′)₂.

### EXAMPLE 3

### Chimaeric B72.3 IgG4 F(ab′)₂ (IgG2 Hinge)

### Construction of F(ab′) Heavy Chain Gene

To construct a hinge modified gene, the chimaeric F(ab) region containing the B72.3VH/IgG4 was isolated as described in Example 2. The IgG2 hinge was assembled by kinase labelling 500 pm of top and bottom strand oligonucleotide and annealing the oligonucleotides by heating to 70°C and cooling to room temperature in the kinase buffer. The hinge fragments were ligated to the 0.7 kbp fragment from JA108 prepared as above, and the CIP′ed 5′ ends were kinased.

### Assembly of Gene in Expression Vector

The chimaeric B72.3 F(ab′) heavy chain gene fragment, assembled as described above was subsequently cloned into the EcoRI/CIP treated vector fragment of JA96 to give TR003. Expression of TR003 in suitable cells with an expression vector capable of producing a useful light chain, for example chimaeric or humanised B72.3, will produce material which will assemble to give F(ab′) and which will on suitable post translational modification in vivo or in vitro give F(ab′)₂.

### EXAMPLE 4

### Modified B72.3 Chimaeric IgG4 F(ab′)₂ (IgG3 Hinge)

### Construction of Modified F(ab′) Heavy Chain Gene

The IgG3 hinge is normally found as a reduplicated 4 exon structure which codes for a repeating sequence of the form
ELKTPLGDTTHTCPRC[PEPKSCDTPPPCPRC]ₙP
where n normally is 3. Hinges of this form can be derived by correct ligation of the following oligonucleotides to the CH1 domain. 
1.    5'TCGACAAGAGAGTTGAGCTCAAAACCCCAC
2.       3'GTTCTCTCAATCGAGTTTTGGGGTGAACC
3.    5'TTGGTGACACACAACTCACACATGCCCACGGTG
4.      3'ACTGTGTTGAGTGTGTACGGGTGCCACGGGT
5. 5'CCCAGAGCCCAAATCTTGTGACACACCTCCCCCGTGCCCACGGTG
6.  3'CTCGGGTTTAGAACACTGTGTGGAGGGGGCACGGGTGCCACGGGGT
7.   5'CCCATGATG
8.   3'ACTACTTAA
By suitable manipulation of the molar ratios of the oligonucleotides, n can be varied from 0 upwards to produce hinges of variable but predictable length and sequence.

To construct the IgG3 hinge, oligonucleotides were assembled by kinase labelling and annealing as in the previous Examples and ligating the relevant oligonucleotides to produce the correctly designed product.Because of the possibility of producing concatamers of the central oligonucleotide pair, the ligation products were cloned into pSP64 at the EcoRI and SalI sites. Miniprep DNA was prepared and cut with EcoRI, 3′ labelled by filing the EcoRI end with ³²P dATP using the Klenow polymerase, recutting the DNA with SalI and examining the length of the cloned inserts of polyacrylamide gels. A clone containing an insert of the required size to code for the IgG3 hinge was identified, the clone was grown up and the hinge insert isolated on a preparative scale. The hinge fragment was ligated to the 0.7 kbp fragment from JA108 prepared as above and the CIP′ed 5′ ends were kinased to enable subsequent recloning.

### Assembly of Gene in Expression Vector

The chimaeric B72.3 F(ab′) heavy chain gene fragment, assembled as described above, was subsequently cloned into the EcoRI/CIP treated vector fragment of JA96 to give TR007. Expression of TR007 in suitable cells with an expression vector capable of producing a useful light chain, for example chimaeric or humanised B72.3, will produce material which will assemble to give F(ab′) and which will on suitable post translational modification in vivo or in vitro give F(ab′)₂.

### EXAMPLE 5

### Modified B72.3 Chimaeric IgG4 F(ab′)₂ (IgG3 2 cys Hinge)

### Construction of F(ab′) Heavy Chain Gene

The IgG3 (2 cys) hinge length variant was assembled by kinase labelling and annealing as above and ligating the relevant oligonucleotides to produce the correct length product. The ligated products were cloned into pSP64 at the EcoRI and SalI sites. Miniprep DNA was prepared and cut with EcoRI, 3′ labelled by filing the EcoRI end with ³²P dATP using the Klenow polymerase, recutting the DNA with SalI and examining the length of the cloned inserts on polyacrylamide gels. A clone containing an insert of the required size to code for the IgG3(2 cys) hinge was identified, the clone was grown up and the hinge insert isolated on a preparative scale. The hinge fragments were ligated to the 0.7 kbp fragment from JA108 and the CIP′ed 5′ ends were kinased to enable subsequent recloning.

### Assembly of Gene in Expression Vector

The chimaeric B72.3 F(ab′) heavy chain gene fragment, assembled as described above, was subsequently cloned into the EcoRI/CIP treated vector fragment of JA96 to give TR004. Expression of TR004 in suitable cells with an expression vector capable of producing a useful light chain, for example chimaeric or humanised B72.3, will produce material which will assemble to give F(ab′) and which will on suitable post translational modification in vivo or in vitro give F(ab′)₂.

### EXAMPLE 6

### Modified B72.3 Chimaeric IgG4 F(ab′)₂ (IgG3 5 cys Hinge)

### Construction of F(ab′) Heavy Chain Gene

The IgG3 (5 cys) hinge length variant was assembled by kinase labelling annealing and ligating the relevant oligonucleotides as above to produce the correct length product. Because of the possibility of producing concatamers of the central oligonucleotide pair, the ligation products were cloned into pSP64 at the EcoRI and SalI sites. Miniprep DNA was prepared and cut with EcoRI 3′ labelled by filing the EcoRI end with ³²P dATP using the Klenow polymerase, recutting the DNA with SalI and examining the length of the cloned inserts on polyacrylamide gels. A clone containing an insert of the required size to code for the IgG3 (5 cys) hinge length variant was identified, the clone was grown up and the hinge insert isolated on a preparative scale. The ligated products were cloned into pSP64 at the EcoRI and Sall sites. The hinge fragments were ligated to the 0.7 kbp fragment from JA108 prepared as above and the CIP′ed ends were kinased to enable subsequent recloning.

### Assembly of Gene in Expression Vector

The chimaeric B72.3 F(ab′) heavy chain gene fragment, assembled as described above, was subsequenty cloned into the EcoRI/CIP treated vector fragment of JA96 to give TR005 in suitable cells with an expression vector capable of producing a useful light chain, for example chimaeric or humanised B72.3, will produce material which will assemble to give F(ab′) and which will on suitable post translational modification in vivo or in vitro give F(ab′)₂.

### EXAMPLE 7

### Modified B72.3 Chimaeric IgG4 F(ab′)₂ (IgG3 8 cys Hinge)

### Construction of F(ab′) Heavy Chain Gene

The IgG3(8 cys) hinge length variant was assembled by kinase labelling annealing and ligating the relevant oligonucleotides as above to produce the correct length product. Because of the possibility of producing concatamers of the central oligonucleotide pair, the ligation products were cloned into pSP64 at the EcoRI and SalI sites. Miniprep DNA was prepared and cut with EcoRI 3′ labelled by filing the EcoRI end with ³²P dATP using the Klenow polymerase, recutting the DNA with SalI and examining the length of the cloned inserts on polyacrylamide gels. A clone containing an insert of the required size to code for the IgG3(8 cys) hinge length variant was identified, the clone was grown up and the hinge insert isolated on a preparative scale. The ligated products were cloned into pSP64 at the EcoRI and Sall sites. The hinge fragments were ligated to the 0.7 kbp fragment from JA108 prepared as above and the CIP′ed ends were kinased to enable subsequent recloning.

### Assembly of Gene in Expression Vector

The chimaeric B72.3 F(ab′) heavy chain gene fragment, assembled as described above, was subsequenty cloned into the EcoRI/CIP treated vector fragment of JA96 to give TR006 in suitable cells with an expression vector capable of producing a useful light chain, for example chimaeric or humanised B72.3, will produce material which will assemble to give F(ab′) and which will on suitable post translational modification in vivo or in vitro give F(ab′)₂.

It will be appreciated that the present invention has been described above by way of illustration only, and that variations or modifications of detail can be made without departing from the scope of the invention.

### References

1. Kohler & Milstein, Nature, 265, 495-497, 1975.
2. Ehrlich, P., Collected Studies on Immunity, 2, John Wiley & Sons, New York, 1906.
3. Levy & Miller, Ann.Rev.Med., 34, 107-116, 1983.
4. Schlom & Weeks, Important Advances in Oncology, 170-192, Wippincott, Philadelphia, 1985.
5. Colcher et al., PNAS, 78, 3199-3203, 1981.
6. Johnson et al., Cancer Res., 46, 850-897, 1986.
7. Stramignoni et al., Int.J.Cancer, 31, 543,552, 1983.
8. Nuti et al., Int.J.Cancer, 29, 539-545, 1982.
9. Thor et al., J.Nat.Cancer Inst., 76, 995-1006, 1986.
10. Thor et al., Cancer Res., 46, 3118-3124, 1986.
11. Maniatis et al., Molecular Cloning, Cold Spring Harbor, New York, 1982.
12. Primrose and Old, Principles of Gene Manipulation, Blackwell, Oxford, 1980.

## Claims

1. An altered antibody molecule (AAM) having a hinge region which has a different number of cysteine residues from that found in the hinge region normally associated with the CH1 domain of the antibody molecule, wherein at least one cysteine residue is capable of forming a heavy chain - heavy chain disulphide bond.

2. The AAM of claim 1, when produced by recombinant DNA technology.

3. The AAM of claim 1 or claim 2, which is a complete antibody molecule or an F(ab')₂ fragment.

4. The AAM of claim 3, which is bispecific.

5. The AAM of any one of claims 1 to 4, having an effector or reporter molecule attached thereto.

6. The AAM of any one of claims 1 to 5, wherein the hinge region comprises a complete hinge region derived from an antibody of different class or subclass from that of the CH1 domain.

7. The AAM of any one of claims 1 to 5, wherein the natural hinge region has been altered by increasing or decreasing the number of cysteine residues.

8. A process for producing a AAM according to claim 2 or any claim dependent thereon, the process comprising:
(a) producing in an expression vector a transcription unit which includes a DNA sequence encoding an antibody heavy chain having a hinge region which has a different number of cysteine residues from that found in the hinge region normally associated with the CH1 domain of the antibody molecule.

9. The process of claim 8, wherein the DNA sequence encoding the hinge region has been altered by site directed mutagenesis.

10. The process of claim 8, wherein the region of the DNA sequence encoding the end of the CH1 domain and the hinge region have been constructed from oligonucleotides.

11. The process of any one of claims 8 to 10, further including the steps of:
(b) transfecting a cell line with the vector; and
(c) culturing the transfected cell line to produce the AAM.

12. The process of claim 11, wherein the cell line is also transformed with a second vector containing a transcription unit having a DNA sequence encoding a complementary light chain-derived polypeptide.

13. The process of claim 11, wherein the vector also contains a DNA sequence encoding a complementary light chain-derived polypeptide.

## Patentansprüche

1. Verändertes Antikörpermolekül (VAM) mit einer Hinge-Region, deren Anzahl an Cysteinresten unterschiedlich ist zu jener, die in der Hinge-Region gefunden wird, welche normalerweise mit der CH1-Domäne des Antikörpermoleküls verbunden ist, wobei mindestens ein Cysteinrest zur Bildung einer schweren Ketteschweren Kette-Disulfidbrücke fähig ist.

2. VAM nach Anspruch 1, hergestellt durch DNA-Rekombinationstechnologie.

3. VAM nach Anspruch 1 oder 2, das ein vollständiges Antikörpermolekül oder ein F(ab')₂-Fragment ist.

4. VAM nach Anspruch 3, das bispezifisch ist.

5. VAM nach einem der vorhergehenden Ansprüche 1 bis 4 mit einem daran gebundenen Effektor- oder Reportermolekül.

6. VAM nach einem der vorhergehenden Ansprüche 1 bis 5, worin die Hinge-Region eine vollständige Hinge-Region umfaßt, die von einem Antikörper einer Klasse oder Subklasse unterschiedlich zu jener der CH1-Domäne stammt.

7. VAM nach einem der vorliegenden Ansprüche 1 bis 5, worin die natürliche Hinge-Region durch Vergrößerung oder Verkleinerung der Anzahl von Cysteinresten verändert worden ist.

8. Verfahren zur Herstellung von VAM nach Anspruch 2 oder eines davon abhängigen Anspruchs, umfassend:
(a) Herstellung einer Transkriptionseinheit in einem Expressionsvektor, die eine DNA-Sequenz umfaßt, welche für eine schwere Kette eines Antikörpers mit einer Hinge-Region codiert, deren Anzahl an Cysteinresten unterschiedlich zu jener in der Hinge-Region ist, die normalerweise mit der CH1-Domäne des Antikörpermoleküls verbunden ist.

9. Verfahren nach Anspruch 8, worin die die Hinge-Region codierende DNA-Sequenz durch spezifische Mutagenese verändert worden ist.

10. Verfahren nach Anspruch 8, worin die Region der DNA-Sequenz, die das Ende der CH1-Domäne und die Hinge-Region codiert, durch Oligonucleotide konstruiert worden ist.

11. Verfahren nach einem der Ansprüche 8 bis 10, ferner umfassend die Schritte:
(b) Transfektion einer Zellinie mit dem Vektor; und
(c) Kultivierung der transfizierten Zellinie zur Produktion von VAM.

12. Verfahren nach Anspruch 11, worin die Zellinie auch mit einem zweiten Vektor transformiert wird, der eine Transkriptionseinheit mit einer DNA-Sequenz aufweist, die für ein komplementäres von einer leichten Kette-stammendes Polypeptid codiert.

13. Verfahren nach Anspruch 11, worin der Vektor auch eine DNA-Sequenz enthält, die für ein komplementäres von einer leichten Kette-stammendes Polypeptid codiert.

## Revendications

1. Molécule d'anticorps modifié (MAM), ayant une région d'articulation possédant un nombre de restes cystéines différent de celui qu'on trouve dans la région d'articulation normalement associée au domaine CH1 de la molécule d'anticorps, molécule dans laquelle au moins un reste cystéine est capable de former un terme de liaison disulfure de chaîne lourde-chaîne lourde.

2. Molécule d'anticorps modifié selon la revendication 1, lorsqu'elle est produite par la technologie de l'ADN (acide désoxyribonucléique) recombinant.

3. Molécule d'anticorps modifié selon la revendication 1 ou la revendication 2, qui est une molécule d'anticorps complet ou un fragment F (ab')₂.

4. Molécule d'anticorps modifié selon la revendication 3, qui est bispécifique.

5. Molécule d'anticorps modifié selon l'une quelconque des revendications 1 à 4, ayant une molécule d'effecteur ou d'un agent de signalisation ou messager qui est fixé sur elle.

6. Molécule d'anticorps modifié selon l'une quelconque des revendications 1 à 5, dans laquelle la région d'articulation comprend une région d'articulation complète provenant d'un anticorps d'une classe ou sous-classe différente de celle du domaine CH1.

7. Molécule d'anticorps modifié selon l'une quelconque des revendications 1 à 5, dans laquelle la région d'articulation naturelle a été altérée ou modifiée par augmentation ou diminution du nombre des restes cystéines.

8. Procédé pour préparer une molécule d'anticorps modifié selon la revendication 2 ou l'une quelconque des revendications qui en dépendant, le procédé comprenant :
(a) la production, dans un vecteur d'expression, d'une unité de transcription qui comprend une séquence d'ADN (acide désoxyribonucléique) codant pour une chaîne lourde d'anticorps ayant une région d'articulation qui possède un nombre de restes cystéines différent de celui trouvé dans la région d'articulation normalement associée au domaine CH1 de cette molécule d'anticorps.

9. Procédé selon la revendication 8 dans lequel la séquence d'ADN qui code pour la région d'articulation a été modifiée par mutagénèse dirigée sur un ou des sites.

10. Procédé selon la revendication 8, dans lequel les régions de la séquence d'ADN codant pour l'extrémité du domaine CH1 et la région d'articulation ont été construites à partir d'oligonucléotides.

11. Procédé selon l'une quelconque des revendications 8 à 10, comprenant en outre les étapes consistant :
(b) à transfecter une lignée de cellules avec le vecteur; et
(c) à cultiver la lignée des cellules transfectées afin de produire la molécule d'anticorps modifié.

12. Procédé selon la revendication 11, dans lequel la lignée des cellules est également transformée à l'aide d'un second vecteur contenant une unité de transcription ayant une séquence d'ADN codant pour un polypeptide dérivant d'une chaîne légère complémentaire.

13. Procédé selon la revendication 11, dans lequel le vecteur contient également une séquence d'ADN codant pour un polypeptide dérivé d'une chaîne légère complémentaire.
